# EUROPÄISCHE PATENTSCHRIFT

(11) **EP 1 610 707 B1**
(45) Veröffentlichungstag und Bekanntmachung des Hinweises auf die Patenterteilung: **08.08.2007**
(21) Anmeldenummer: 04723596.5
(22) Anmeldetag: 26.03.2004
(51) Int. Cl.: A61C 13/00, G06F 19/00

(54) **CAD SYSTEM FÜR DENTALPROTHESEN**
CAD-SYSTEM FOR DENTAL PROSTHESIS
SYSTEME DE CONCEPTION ASSISTEE PAR ORDINATEUR POUR PROTHESES DENTAIRES

(30) Priorität: 26.03.2003 DE 10313691
(43) Veröffentlichungstag der Anmeldung: 04.01.2006
(73) Patentinhaber: 3M ESPE AG, 82229 Seefeld (DE)
(72) Erfinder: KRAEMER, Michael, 86899 Landsberg (DE); HERTLEIN, Guenter, 82229 Seefeld (DE); BURGER, Bernd, 82239 Alling (DE)
(74) Vertreter: Vossius & Partner
(86) Internationale Anmeldenummer: PCT/EP2004/003259
(87) Internationale Veröffentlichungsnummer: WO 2004/084757

(56) Entgegenhaltungen:
- EP-A- 1 088 620
- WO-A-03/007834
- US-A1- 2002 102 521

## Beschreibung

Die vorliegende Erfindung betrifft die Herstellung von Zahnersatz, genauer gesagt ein CAD-System für die Bearbeitung von Daten über die dreidimensionale Gestalt einer dentalen Prothese.

Der Begriff "dentale Prothese" ist hier im weitesten Sinne zu verstehen und soll alle Arten von Zahnersatz umfassen, wie beispielsweise Brücken, Implantate und Zahnprothesen im engeren Sinne, aber auch Teile solcher dentalen Prothesen, wie beispielsweise Kronengerüste und Brückengerüste, auf die erst noch eine Verblendung aufgebracht werden muss, um die fertige Krone oder Brücke zu erhalten, sowie einzelne Brückengerüst- oder Brückenglieder.

Die Erfindung umfasst daher beispielsweise nicht nur zweigliedrige Brückengerüste, bei denen die beiden Glieder über einen Verbinder verbunden sind, sondern auch drei- und mehrgliedrige Brückengerüste, bei denen jeweils zwei benachbarte Glieder über einen Verbinder verbunden sind. Die Glieder können nach Bedarf Anker, Zwischenglieder oder Freiendglieder sein: ein Anker ist wie eine Krone auf einem als Brückenpfeiler dienenden Zahnstumpf befestigt, ein Zwischenglied ist zwischen zwei Gliedern und nicht an einem Brückenpfeiler befestigt, und ein Freiendglied ist an nur einem Glied und nicht an einem Brückenpfeiler befestigt. Auch diese Glieder werden von der Erfindung umfasst.

WO 03/007834 betrifft ein Verfahren zur Herstellung von auf einem Zahnstumpf zu befestigenden Zahnersatz aus einem dreidimensional vermessenen und digitalisierten Positivmodel. Das beschriebene Verfahren weist die folgenden Schritte auf:
- Einlesen der Daten des digitalisierten Positivmodels;
- Beaufschlagen der Innenfläche des Positivmodels mit einem vorgegebenen Offsetwert zur Bildung eines Spaltes zwischen späterem Zahnersatz und Zahnstumpf; und
- Berechnung eines Programms für eine Bearbeitung eines Rohlings durch eine Werkzeugmaschine zur Herstellung des Zahnersatzes.

Bei diesem Verfahren erfolgt für eine besonders hohe Sicherheit gegen Bruch des Zahnersatzes bei späterer Benutzung durch den Patienten ein Berechnen der Wandstärke des Datenmodels durch Vergleichen der Daten der Innenfläche des Positivmodels mit den Daten der Außenfläche des Positivmodels und Vergleichen des Wertes der Wandstärke mit einem Wandstärkenmindestwert, der entweder durch eine Eingabe des Zahnarztes oder Zahntechnikers bestimmt oder die Programmierung fest voreingestellt sein kann.

Es ist bekannt, dass die Verarbeitung von Daten über die dreidimensionale Gestalt eines Kronen- oder Brückengerüstes mit Hilfe eines CAD-Systems erfolgen kann, das Teil eines CIM-Systems ist, das unter dem Namen LAVA von der 3M ESPE AG (Seefeld, Deutschland) für die Herstellung von keramischen Kronen- und Brückengerüsten angeboten wird. Bei diesem bekannten LAVA-System ist das CAD-System zum Einen an einen optischen Scanner und zum Anderen an eine NC-Fräsmaschine angeschlossen. Der Scanner erfasst die dreidimensionale Oberfläche eines Gebissabdruckes und übergibt die erfassten Daten an das CAD-System. Mit dem CAD-System kann der Anwender diese Oberflächendaten nach Wunsch bearbeiten oder modifizieren, um die dreidimensionale Gestalt des Kronen- oder Brückengerüstes zu entwerfen, und dann die entsprechenden Gestaltdaten an die NC-Fräsmaschine schicken. Die NC-Fräsmaschine bearbeitet schließlich einen Keramikrohling aus Zirkonia oder Zirkoniumdioxid in möglichst genauer Übereinstimmung mit den Gestaltdaten.

Die mit diesem bekannten CAD-System entworfenen Kronengerüste weisen eine gleichförmige Dicke auf. Die entsprechenden Gestaltdaten für das Gerüst werden vom System automatisch aus den vom Scanner erhaltenen Eingabedaten, die ja die dreidimensionale Oberfläche des für die Krone präparierten Zahnstumpfes darstellen, wie folgt errechnet: Diese Oberflächen-Eingabedaten werden kopiert, und diese kopierten Daten werden dann relativ zu den Originaldaten derart auswärts skaliert, dass für jeden Punkt der Originaloberfläche der normale Abstand, also der Abstand in Richtung der Oberflächennormale, zur äußeren Kopieoberfläche einen vorgegebenen Festwert hat. Dieser Festwert stellt also die gleichförmige Dicke des Gerüstes dar und wird so ausgewählt, dass das Gerüst die gewünschte Stabilität hat, die es benötigt, um die Belastungen bei der späteren Fräsbearbeitung und, nach der Fertigstellung und dem Aufbringen auf den Zahnstumpf, beim Kauen aushalten zu können.

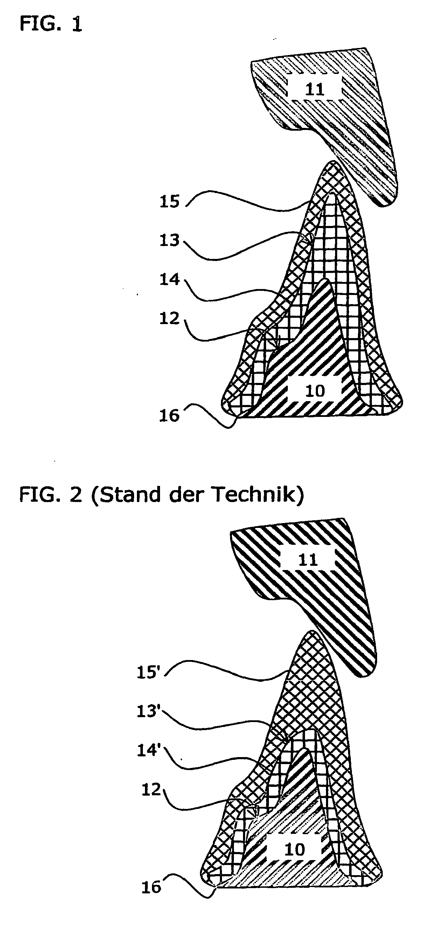

Diese mit dem bekannten CAD-System entworfenen Kronengerüste mit gleichförmiger Dicke sind im Allgemeinen sehr brauchbar, führen aber bei besonderen Fällen zu Problemen, die im Folgenden an Hand der FIG. 2 beispielhaft näher erläutert werden.

In der FIG. 2 sind ein unterer Schneidezahn, genauer gesagt sein bis zu einem Präparationsrand 16 präparierter Stumpf 10, und der obere Gegenzahn 11 im Querschnitt dargestellt. In dem dargestellten problematischen Fall musste der Zahnarzt den inzisalen Teil des unteren Zahnes ziemlich weit entfernen, so dass nun eine große Lücke zwischen Zahnstumpf 10 und Gegenzahn 11 klafft. Mit dem bekannten CAD-System werden nun, ausgehend von den Originaldaten der Stumpfoberfläche 12 oberhalb des Präparationsrandes 16, die skalierten Kopiedaten für die äußere Gerüstoberfläche 13'errechnet, die in gleichförmigem Abstand zur Stumpfoberfläche 12 liegt, so dass das Gerüst 14' eine gleichförmige Dicke hat.

Wie in der FIG. 2 gut zu erkennen ist, muss die vom Zahntechniker auf das Gerüst 14' aufgebrachte Verblendung 15' die verbleibende, durch das Gerüst 14'kaum geschlossene Lücke zum Gegenzahn 11 schließen. Die Dicke der Verblendung sollte aber einen gewissen Maximalwert nicht überschreiten, da sonst die Stabilität der Verblendung zu stark sinkt.

Bei diesem Problem setzt die Erfindung mit einem ersten Aspekt an, indem sie vorschlägt, das Gerüst im inzisalen Bereich dicker als bisher üblich auszubilden. Dies ist in der FIG. 1 gut zu erkennen.

Dies kann beispielsweise durch eine sogenannte "globale" Modifikation der bekannten äußeren Gerüstoberfläche 13, die zu dem Gerüst 14 mit gleichförmiger Dicke gehört, erreicht werden, indem diese auf bekanntem Weg errechnete Kopieoberfläche in wenigstens zwei Raumachsen unterschiedlich skaliert wird. Bei dem in der FIG. 1 dargestellten Gerüst 14 wurde die die äußere Gerüstoberfläche 13 darstellende Kopieoberfläche beispielsweise in vertikaler Richtung stärker vergrößert als in saggitaler Richtung.

Als Folge ist im inzisalen Bereich die Verblendung 15 der FIG. 1 deutlich dünner als die Verblendung 15' der FIG. 2, so dass sie eine höhere Stabilität hat. Außerdem kann der Zahntechniker die Verblendung 15 der FIG. 1 schneller herstellen, da er weniger Material auf das Gerüst 14 aufbringen muss.

Die globale Modifikation gemäß der Erfindung kann von dem CAD-System derart durchgeführt werden, dass der untere Präparationsrand 16 nicht geändert wird. Dies ist für einen exakten Sitz des Gerüstes 14 auf dem Stumpf 10 wichtig. Außerdem kann die Skalierung in einer bestimmten Raumachse nicht nur mit konstantem Skalierungsfaktor erfolgen, sondern auch mit einem variablen Skalierungsfaktor, der beispielsweise vom Abstand zum Präparationsrand 16 abhängt. So kann beispielsweise eine trapezoidale Skalierungsfunktion für die vertikale Achse und/oder die saggitale Achse verwendet werden, so dass die Kopie- beziehungsweise Gerüstoberfläche 13 am stärksten im inzisalen Bereich verzerrt wird. Dadurch kann die natürliche Zahnform sehr gut angenähert werden.

Es ist auch möglich, die Skalierung für den positiven und den negativen Teil einer Raumachse unterschiedlich zu wählen, um so beispielsweise in distaler Richtung eine andere Verzerrung als in mesialer Richtung zu erzielen.

Die Skalierung kann durch Zahleneingabe über eine Tastatur und/oder mit einer Maus eingestellt werden.

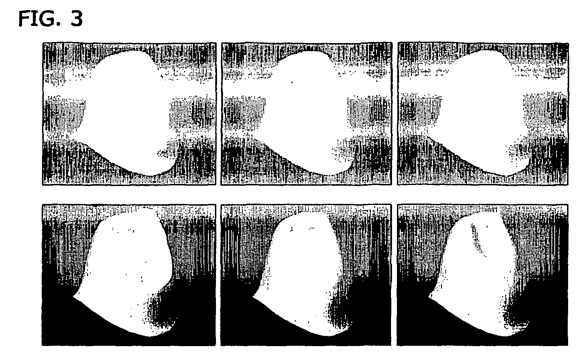

Da eine globale Modifikation nicht alle möglichen optimal Fälle abdecken kann, sieht die Erfindung in einem zweiten Aspekt eine sogenannte "lokale" Modifikation vor. Diese kann beispielsweise einem herkömmlichen Wachsmesser nachempfunden sein, um dem Zahntechniker die Anwendung zu erleichtern. Wie in der Bilderfolge der FIG. 3 dargestellt, muss der Anwender Bereiche der Oberfläche mit der Maus markieren (in der FIG. 3 rot dargestellt), die dann mit zuvor gesetzten Parametern modifiziert werden. Diese Parameter umfassen zumindest den Durchmesser und die Stärke der lokalen Modifikation. Unter Stärke wird hier die Dicke des aufgetragenen oder entfernten Gerüstmaterials verstanden. Es kann auch eine sogenannte Temperatur verwendet werden, die definiert, wie stark die Oberfläche während der Modifikation geglättet wird.

Bei jeder Modifikation müssen bestimmte Bedingungen erfüllt sein, damit die minimalen Stabilitätsanforderungen für die Prothese erhalten bleiben. So muss beispielsweise ein Gerüst eine minimale Wandstärke haben, um Brüche zu vermeiden. Dies kann gemäß einem dritten Aspekt der vorliegenden Erfindung dadurch überwacht werden, dass eine zusätzliche Kontrolloberfläche erzeugt wird, die die minimalen Stabilitätsanforderungen erfüllt und zusammen mit der aktuellen Oberfläche des Gerüsts dargestellt wird.

In einem vierten Aspekt betrifft die vorliegende Erfindung ein Verfahren zur Verarbeitung von Daten über die dreidimensionale Gestalt einer dentalen Prothese (14), welches Verfahren die Schritte aufweist, dass:
a) Eingabedaten bereitgestellt werden, die eine dreidimensionale Oberfläche des für die Prothese (14) präparierten Zahnstumpfes (10) darstellen;
b) minimale Stabilitätsanforderungen für die Prothese (14) bereitgestellt werden;
c) aus den Eingabedaten Kontrolldaten erzeugt werden, die eine Kontrolloberfläche darstellen, die die minimalen Stabilitätsanforderungen erfüllt;
d) Gestaltdaten erzeugt werden, die die dreidimensionale Gestalt der Prothese (14) darstellen;
e) die Gestalt der Prothese (14) zusammen mit der Kontrolloberfläche dargestellt wird.

In Schritt a können die Eingabedaten beispielsweise von einem Scanner, der die dreidimensionale Oberfläche eines Gebissabdruckes erfasst, oder von einem Intraoral-Scanner, der die dreidimensionale Oberfläche einer Gebisssituation im Mund eines Patienten erfasst, bereitgestellt werden.

In Schritt b können die Stabilitätsanforderungen automatisch, beispielsweise mit Hilfe eines Computers, und/oder manuell durch den Anwender bereitgestellt werden.

In Schritt c können die Kontrolldaten automatisch, beispielsweise mit Hilfe eines Computers, und/oder manuell durch den Anwender erzeugt werden.

In Schritt d können die Gestaltdaten automatisch, beispielsweise mit Hilfe eines Computers, und/oder manuell durch den Anwender erzeugt werden.

In Schritt e kann die Darstellung beispielsweise mit Hilfe eines Monitors erfolgen.

Die Reihenfolge der Schritte kann nach Wunsch oder Bedarf gewählt werden. So kann beispielsweise Schritt d vor, gleichzeitig oder nach Schritt c erfolgen. Auch kann in Schritt e beispielsweise zuerst die Kontrolloberfläche allein und danach die Gestalt der Prothese zusätzlich, beispielsweise durch Einblendung, dargestellt werden, allerdings ist auch die umgekehrte Reihenfolge oder ein gleichzeitiger Darstellungsbeginn möglich.

Es kann vorgesehen sein, dass dieses Verfahren die weiteren Schritten aufweist, dass:
f) die Gestaltdaten modifiziert werden;
g) die aktuelle Gestalt der Prothese (14), die den modifizierten Gestaltdaten entspricht, zusammen mit der Kontrolloberfläche dargestellt wird.

In Schritt f können die Gestaltdaten global, beispielsweise gemäß der weiter oben stehenden Definition, und/oder lokal, beispielsweise gemäß der weiter oben stehenden Definition, und/oder automatisch, beispielsweise mit Hilfe eines Computers, und/oder manuell durch den Anwender modifiziert werden.

In Schritt g kann beispielsweise zuerst die Kontrolloberfläche allein und danach die aktuelle Gestalt der Prothese zusätzlich, beispielsweise durch Einblendung, dargestellt werden, allerdings ist auch die umgekehrte Reihenfolge oder ein gleichzeitiger Darstellungsbeginn möglich.

Es kann vorgesehen sein, dass in Schritt d die Gestaltdaten aus den Eingabedaten erzeugt werden.

Es kann vorgesehen sein, dass die Gestaltdaten global derart modifiziert werden, dass ein vorgegebener Präparationsrand (16) unverändert bleibt.

Es kann vorgesehen sein, dass die Kontrolloberfläche die minimalen Stabilitätsanforderungen genau erfüllt.

Es kann vorgesehen sein, dass das Verfahren gemäß der Erfindung mit Hilfe eines Computerprogramms durchgeführt wird.

In einem fünften Aspekt betrifft die vorliegende Erfindung eine Datenverarbeitungsanlage zur Ausführung des Verfahrens gemäß der Erfindung, mit:
- einem Eingabegerät für die in dem Verfahren benötigten Daten;
- einer an das Eingabegerät angeschlossenen Zentraleinheit, in dem ein Programm zur Verarbeitung der Daten gemäß dem Verfahren abläuft;
- einem an die Zentraleinheit angeschlossenen Ausgabegerät für die Gestalt der Prothese (14) und die Kontrolloberfläche.

In einem sechsten Aspekt betrifft die vorliegende Erfindung ein Computerprogramm, das derart ausgebildet ist, dass es das Verfahren gemäß der Erfindung ausführt.

In einem siebten Aspekt betrifft die vorliegende Erfindung ein Computerprogramm, das, wenn es auf einem Computer läuft, das Verfahren gemäß der Erfindung ausführt.

In einem achten Aspekt betrifft die vorliegende Erfindung ein Computerprogramm, das Kommandos aufweist, die das Verfahren gemäß der Erfindung ausführen.

In einem neunten Aspekt betrifft die vorliegende Erfindung ein Computerprogramm, das das Verfahren gemäß der Erfindung implementiert.

In einem zehnten Aspekt betrifft die vorliegende Erfindung einen Datenträger, auf dem ein Computerprogramm gemäß der Erfindung gespeichert ist. Der Datenträger kann beispielsweise eine Floppy-Disk, ein Magnetband, eine CD, eine DVD, ein Memory-Stick, eine Festplatte, ein RAM-Baustein, oder ein ROM- Baustein sein.

Die vorliegende Erfindung wurde nun unter Bezugnahme auf verschiedene ihrer Ausführungsformen beschrieben. Es wird für den Fachmann klar sein, dass an den beschriebenen Ausführungsformen viele Änderungen vorgenommen werden können, ohne vom Umfang der vorliegenden Erfindung abzuweichen. Daher soll der Umfang der vorliegenden Erfindung nicht auf die in dieser Anmeldung beschriebenen Strukturen beschränkt sein, sondern nur durch die Strukturen, die durch den Wortlaut der Ansprüche beschrieben sind, sowie durch die Äquivalente jener Strukturen.

### BEZUGSZEICHENLISTE

- 10: Stumpf
- 11: Gegenzahn
- 12: Stumpfoberfläche
- 13, 13': Äußere Gerüstoberfläche
- 14, 14': Gerüst
- 15, 15': Verblendung
- 16: Präparationsrand

## Patentansprüche

1. Verfahren zur Verarbeitung von Daten über die dreidimensionale Gestalt einer dentalen Prothese (14), welches Verfahren die Schritte aufweist, dass:
a) Eingabedaten bereitgestellt werden, die eine dreidimensionale Oberfläche des für die Prothese (14) präparlerten Zahnstumpfes (10) darstellen;
b) minimale Stabilitätsanforderungen für die Prothese (14) bereitgestellt werden;
c) aus den Eingabedaten Kontrolldaten erzeugt werden, die eine Kontrolloberfläche darstellen, die die minimalen Stabilitätsanforderungen erfüllt;
d) Gestaltdaten erzeugt werden, die die dreidimensionale Gestalt der Prothese (14) darstellen;
e) die Gestalt der Prothese (14) zusammen mit der Kontrolloberfläche auf einem Monitor dargestellt wird,
wobei
f) die Gestaltdaten anhand eines visuellen Vergleichs der dargestellten Gestaltdaten mit der dargestellten Kontrolloberfläche durch den Anwender modifiziert werden, um die minimalen Stabilitäts anforderungen zu erfüllen; und
g) die aktuelle Gestalt der Prothese (14), die den modifizierten Gestaltdaten entspricht, zusammen mit der Kontrolloberfläche dargestellt wird.

2. Verfahren nach Anspruch 1, wobei in Schritt d die Gestaltdaten aus den Eingabedaten erzeugt werden.

3. Verfahren nach einem der vorhergehenden Ansprüche, wobei die äußer Oberfläche der Prothese (14) in mindestens zwei Ramachsen unterschiedlich skaliert wird, derart, dass ein vorgegebener Präparationsrand (16) dabei unverändert bleibt.

4. Verfahren nach einem der vorhergehenden Ansprüche, wobei die Kontrolloberfläche die minimalen Stabilitätsanforderungen genau erfüllt.

5. Verfahren nach einem der vorhergehenden Ansprüche, das mit Hilfe eines Computerprogramms durchgeführt wird.

6. Datenverarbeitungsanlage zur Ausführung des Verfahrens nach einem der vorhergehenden Ansprüche, mit:
- einem Eingabegerät für die in dem Verfahren benötigten Daten;
- einer an das Eingabegerät angeschlossenen Zentraleinheit, in dem ein Programm zur Verarbeitung der Daten gemäß dem Verfahren abläuft;
- einem an die Zentraleinheit angeschlossenen Ausgabegerät für die Gestalt der Prothese (14) und die Kontrolloberfläche.

7. Computerprogramm, das derart ausgebildet ist, dass es das Verfahren nach einem der Ansprüche 1-5 ausführt.

8. Computerprogramm, das, wenn es auf einem Computer läuft, das Verfahren nach einem der Ansprüche 1-5 ausführt.

9. Computerprogramm, das Kommandos aufweist, die das Verfahren nach einem der Ansprüche 1-5 ausführen.

10. Computerprogramm, das das Verfahren nach einem der Ansprüche 1-5 implementiert.

11. Datenträger, auf dem ein Computerprogramm nach einem der Ansprüche 7-10 gespeichert ist.

## Claims

1. Method for processing data concerning the three-dimensional shape of a dental prosthesis (14), which method comprises the steps of:
a) providing input data representing a three-dimensional surface of the tooth stump (10) prepared for the prosthesis (14);
b) providing minimum stability requirements for the prosthesis (14);
c) using the input data to produce control data, which represent a control surface that meets the minimum stability requirements;
d) producing shape data, which represent the three-dimensional shape of the prosthesis (14);
e) displaying the shape of the prosthesis (14) together with the control surface on a monitor,
wherein
f) the shape data are modified by the user on the basis of a visual comparison of the displayed shape data with the displayed control surface, in order to meet the minimum stability requirements; and
g) the current shape of the prosthesis (14), which corresponds to the modified shape data, is displayed together with the control surface.

2. Method according to Claim 1, wherein, in step d, the shape data are produced from the input data.

3. Method according to one of the preceding claims, wherein the outer surface of the prosthesis (14) is differently scaled in at least two spatial axes, in such a way that a predetermined preparation edge (16) remains unchanged.

4. Method according to one of the preceding claims, wherein the control surface precisely meets the minimum stability requirements.

5. Method according to one of the preceding claims, which is carried out with the aid of a computer program.

6. Data-processing system for implementing the method according to one of the preceding claims, comprising:
- an input device for the data required in the method;
- a central processing unit, which is connected to the input device and in which a program for processing the data according to the method runs;
- an output device, connected to the central processing unit, for the shape of the prosthesis (14) and the control surface.

7. Computer program, which is designed in such a way that it executes the method according to one of Claims 1-5.

8. Computer program, which, when it runs on a computer, executes the method according to one of Claims 1-5.

9. Computer program, which has commands that execute the method according to one of Claims 1-5.

10. Computer program, which implements the method according to one of Claims 1-5.

11. Data carrier, on which a computer program according to one of Claims 7-10 is stored.

## Revendications

1. Procédé de traitement de données concernant la forme tridimensionnelle d'une prothèse dentaire (14), lequel procédé présente les étapes consistant à :
a) élaborer des données de saisie représentant une surface tridimensionnelle du moignon de dent (10) préparé pour la prothèse (14) ;
b) élaborer des prescriptions minimales de stabilité pour la prothèse (14);
c) générer à partir des données de saisie des données de contrôle représentant une surface de contrôle remplissant les prescriptions minimales de stabilité ;
d) générer des données de forme représentant la forme tridimensionnelle de la prothèse (14) ;
e) représenter la forme de la prothèse (14) avec la surface de contrôle sur un moniteur,
dans lequel
f) les données de forme sont modifiées par l'utilisateur à partir d'une comparaison visuelle des données de forme représentées et de la surface de contrôle représentée afin de remplir les prescriptions minimales de stabilité et
g) la forme réelle de la prothèse (14) correspondant aux données de forme modifiées est représentée avec la surface de contrôle.

2. Procédé selon la revendication 1, dans lequel les données de forme sont générées en étape d à partir des données de saisie.

3. Procédé selon l'une quelconque des revendications précédentes, dans lequel la surface extérieure de la prothèse (14) est échelonnée différemment dans au moins deux axes spatiaux de manière à ce qu'un bord de préparation prescrit (16) reste alors inchangé.

4. Procédé selon l'une quelconque des revendications précédentes, dans lequel la surface de contrôle remplit précisément les prescriptions minimales de stabilité.

5. Procédé selon l'une quelconque des revendications précédentes, qui est réalisé à l'aide d'un programme informatique.

6. Installation de traitement de données pour exécuter le procédé selon l'une quelconque des revendications précédentes, comportant :
- un appareil de saisie pour les données nécessaires au procédé ;
- une unité centrale raccordée à l'appareil de saisie, dans laquelle se déroule un programme de traitement des données selon le procédé ;
- un appareil d'édition raccordé à l'unité centrale pour la forme de la prothèse (14) et la surface de contrôle.

7. Programme informatique qui est réalisé de manière à exécuter le procédé selon l'une quelconque des revendications 1 à 5.

8. Programme informatique qui, lorsqu'il fonctionne sur un ordinateur, exécute le procédé selon l'une quelconque des revendications 1 à 5.

9. Programme informatique comportant des commandes qui exécutent le procédé selon l'une quelconque des revendications 1 à 5.

10. Programme informatique qui met en oeuvre le procédé selon l'une quelconque des revendications 1 à 5.

11. Support de données sur lequel un programme informatique selon l'une quelconque des revendications 7 à 10 est sauvegardé.
